# EUROPEAN PATENT APPLICATION

(11) **EP 4 529 920 A1**
(43) Date of publication of application: **02.04.2025**
(21) Application number: 24199257.7
(22) Date of filing: 09.09.2024
(51) Int. Cl.: A61K 9/12, A61K 8/14

(54) **NOVEL LIPOSOMAL FORMULATIONS AND A PROCESS FOR THEIR PREPARATION**

(30) Priority: 12.09.2023 SI 202300123
(71) Applicant: Univerza V Ljubljani, 1000 Ljubljana (SI)
(72) Inventor: Poklar Ulrih, Natasa, 1000 Ljubljana (SI); Kejzar, Jan, 1351 Brezovica pri Ljubljani (SI); Osojnik Crnivec, Ilja Gasan, 1411 Izlake (SI)
(74) Representative: Macek, Gregor

(57) **Abstract**

The subject of the invention are new liposomal formulations and a process for their preparation. The liposomal formulations exhibit exceptional physicochemical stability and allow for the preparation of liposomes of various sizes using thin film, sonication, extrusion, and gel swelling methods. The liposomal formulations according to the invention have a lipid bilayer composed of 40 to 80 mol % phospholipids, 20 to 60 mol % cholesterol, and 0.5 to 50 mol % of a polar lipid extract from the archaeon *Aeropyrum pernix* K1. These methods produce liposomes that are colloidally stable and remain morphologically unchanged over extended periods (>30 days).

## Description

### Field of the invention

The subject of the invention are new liposomal formulations, where the lipid bilayer of the liposomal formulations, i.e., liposomes (vesicles), includes an addition of various proportions of archaeal lipids alongside conventional phospholipids and cholesterol, as well as the processes for their preparation. The addition of archaeal lipids to the mixture of phospholipids and cholesterol affects the properties of the liposomes. The influence of archaeal lipids is highly pronounced even with minimal additions. By incorporating a portion of archaeal lipids into the liposomal formulations, specifically into their lipid bilayer, the following is achieved: (i) homogeneous size distribution and facilitated formation of liposomes; (ii) a reduction in the zeta potential and, consequently, improved stability of the colloidal liposome solutions; (iii) increased fluidity of the lipid bilayer; and (iv) prolonged stability of the liposomes even at elevated temperatures, confirmed by unchanged morphological properties and calcein release studies.

Preferably, the lipid bilayer of the liposome is based on egg sphingomyelin and cholesterol.

According to the invention, archaeal ether lipids, extracted from the thermophilic archaeon *Aeropyrum pernix* K1, are used. The liposomal formulations of the invention include archaeal lipids, specifically polar lipid extracts obtained through a cost-effective in-house process of cultivating archaeal biomass. This is also a novelty, as comparable components are either synthetic or involve cost-intensive processes of archaeal cultivation, which hampers the economic feasibility of the commercial process.

Single-layer liposomes of various sizes can be prepared, where single-layer means they have a single lipid bilayer, specifically:
- small single-layer (unilamellar) liposomes (vesicles) (diameter up to 100 nm; Small Unilamellar Vesicles - SUV);
- large single-layer liposomes (vesicles) (diameter 100-1000 nm; Large Unilamellar Vesicles - LUV);
- giant single-layer liposomes (vesicles) (diameter over 1 µm; Giant Unilamellar Vesicles - GUV).

Small and large single-layer liposomes are primarily used for delivering biologically active ingredients to the target site.

### Technical challenge

The main drawback of conventional liposomes or liposomal formulations is their poor stability and, consequently, their reduced ability to deliver active ingredients to the target site¹. Additionally, the manufacturing of liposomes is a precise and complex process with numerous steps that significantly impact the final size, stability, and functionality of the end product².

In our previous research, we have already demonstrated the potential of *A. pernix* archaeal lipids in single-component liposomes, which exhibited good morphological characteristics and physicochemical stability^{3,4}. However, a primary issue with these liposomes made from 100 mol % archaeal lipids was, on one hand, their poor stability in biological systems⁵, and, on the other hand, the inability to release their cargo after cellular uptake of entire liposomes (demonstrated across various cell lines)⁶.

The standalone use of archaeal lipids for liposome formation is also not economically justified.

### State of the art

Despite extensive research and transfer attempts to clinical applications, there are fewer than 20 registered liposome-based drugs on the market⁷. A few drugs utilizing a delivery system based on sphingomyelin and cholesterol are also patented. Although existing systems are therapeutically effective, they suffer from inadequate physicochemical stability. An example is the product Marqibo^{®}, provided as a three-vial kit that separately contains (i) the active ingredient vincristine sulfate, (ii) sphingomyelin and cholesterol as liposome components, and (iii) a working buffer. Preparing the encapsulated active ingredient in liposomes for final therapeutic use requires a lengthy process (26 steps) under specific conditions⁸. To ensure the stability of this formulation, the preparation of the Marqibo^{®} liposomal formulation must be completed no more than 12 hours before use. Unlike this example, our invention with the addition of archaeal lipids simplifies the liposome preparation process and extends the stability of the delivery system.

The basic formulation of sphingomyelin and cholesterol appears in numerous patents. For example, patent document EP 2266537 A2 relates to cancer treatment with the active ingredient vincristine, encapsulated in liposomes. Patent document US 2023/0028179 describes the treatment of viral infections using liposomes based on sphingomyelin and cholesterol. Unlike our invention, these patents do not mention the addition of archaeal lipids to the liposomal base in question. We found no inventions in patent databases that add archaeal lipids to a sphingomyelin and cholesterol formulation. Additionally, archaeal lipids (from *A. pernix* K1) are mentioned exclusively in patent document EP 2518138 A1, which relates to a different application.

The invention is described in more detail below and is presented with images and examples of embodiments, where the images show:

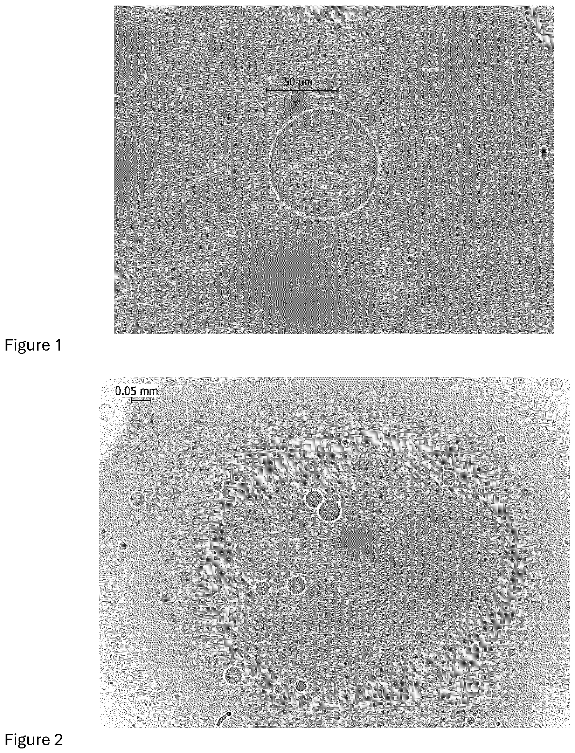

Figure 1: Giant liposome (GUV) with a 2% content of archaeal lipids, prepared using the gel swelling method. Photographed with a light microscope (400x magnification, Leica DM750, Leica Microsystems Ltd., China).
Figure 2: A cluster of giant liposomes (GUV) with a 2% content of archaeal lipids, prepared using the gel swelling method. Photographed with a light microscope (40x magnification, Leica DM750, Leica Microsystems Ltd., China).
Figure 3: Uptake of large liposomes (LUV) with a 2% content of archaeal lipids, loaded with 80 mM calcein, into CHO (Chinese Hamster Ovary) cells. Photographed with an inverted microscope with a fluorescence module (400x magnification, Leica DM IL LED, Leica Ltd., China).

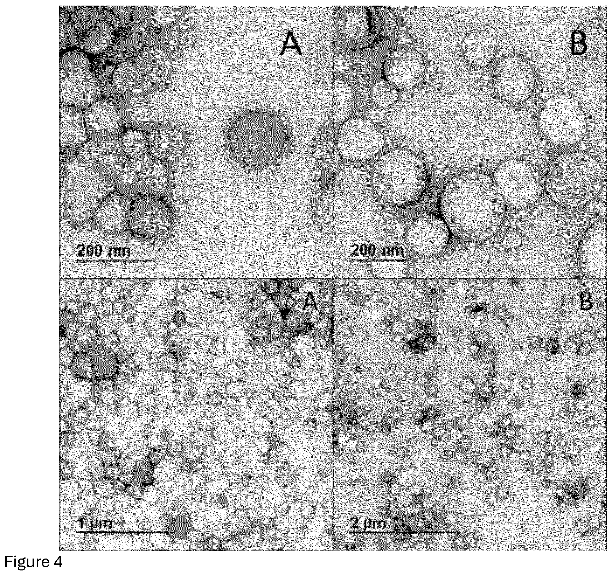

Figure 4: Comparison of the morphology of large liposomes (LUV, prepared by extrusion through a 100 nm membrane) without archaeal lipids (A) and with a 5% addition of archaeal lipids (B). Samples were applied to copper grids (400 mesh, SPI, USA) with a layer of Formvar support film, pre-sprayed with carbon and made more hydrophilic by the glow discharge surface modification method (EM ACE200 coater, Leica Microsystems, Austria). After 30 seconds of incubation, the sample was contrasted with a 1% aqueous solution of uranyl acetate. Sample examination and documentation of results were carried out on a Philips CM 100 transmission electron microscope (Amsterdam, Netherlands) equipped with an Orius SC200 digital camera and Digital Micrograph software (Gatan Inc., Washington, USA), or on a TALOS L120C transmission electron microscope (ThermoFisher Scientific, Waltham, MA, USA) equipped with a Ceta 16M digital camera and Velox software.

In the context of this application, the terms liposomal formulation, liposome, and vesicle are used equivalently and interchangeably.

According to the invention, the lipid bilayer of the liposomal formulation consists of phospholipids, cholesterol, and archaeal lipids, specifically from the polar lipid extract of the archaeon *Aeropyrum pernix* K1. The molar fraction of phospholipids is between 40 and 80 mol %, cholesterol between 20 and 60 mol %, and the polar lipid extract from *Aeropyrum pernix* K1 between 0.5 and 50 mol %.

The conventional phospholipid is selected from phosphatidylcholine, phosphatidylserine, phosphatidylethanolamine, or sphingomyelin, with acyl side chains containing 8-24 carbon atoms.

Preferably, the phospholipid is sphingomyelin, with acyl side chains containing 8-24 carbon atoms, which may be of natural origin, such as egg or brain sphingomyelin, or synthetic.

Preferably, the polar lipid extract from the archaeon *Aeropyrum pernix* K1 consists of two fractions of polar lipids, specifically 91 mol % of 2,3-di-O-sesterterpanyl-*sn*-glycerol-1-phospho-1'-(2'-*O*-α-D-glucosyl)-myo-inositol (C_{25,25}-archaetidyl(glucosyl)inositol) and 9 mol % of 2,3-di-O-sesterterpanil-*sn*-glycerol-1-phospho-myo-inositol (C_{25,25}-archaetidylinositol).

Table 1 shows various liposomal formulations according to the invention.

**Table 1: Calculated molar fractions of sphingomyelin, cholesterol, and archaeal lipids in selected examples of the liposomal formulation (2, 5, 15, 25, 35, and 45 mol % addition of archaeal lipids).**

| **ARCHAEAL LIPIDS FRACTION** | **ARCHAEAL LIPIDS (mol %)** | **EGG SPHINGOMYELIN (mol %)** | **CHOLESTEROL (mol %)** |
|---|---|---|---|
| **2 mol %** | 2 | 58.8 | 39.2 |
| **5 mol %** | 5 | 57 | 38 |
| **15 mol %** | 15 | 51 | 34 |
| **25 mol %** | 25 | 45 | 30 |
| **35 mol %** | 35 | 39 | 26 |
| **45 mol %** | 45 | 33 | 22 |

The addition of archaeal lipids in the presented liposomal formulations demonstrates significant improvements in the formulation even with the addition of 2 mol %. Implementation examples show that the addition of archaeal lipids to the egg sphingomyelin and cholesterol mixture enables the formation of smaller vesicles with a substantially lower polydispersity index compared to formulations without added archaeal lipids (Table 2).

**Table 2: Average diameter, polydispersity index, and zeta potential of small unilamellar vesicles (SUV) prepared by sonication. The parameters were measured using dynamic light scattering and phase analysis with the ZetaSizer Nano device (Malvern Panalytical, Malvern, UK).**

| **ARCHAEAL LIPIDS FRACTION** | **MEAN DIAMETER (nm)** | **POLYDISPERSITY INDEX** | **ZETA POTENCIAL (mV)** |
|---|---|---|---|
| 0 mol % | 152 | 0.388 | -12 |
| 2 mol % | 86 | 0.191 | -29 |
| 5 mol % | 81 | 0.185 | -37 |
| 15 mol % | 84 | 0.194 | -47 |
| 25 mol % | 88 | 0.171 | -47 |
| 35 mol % | 80 | 0.185 | -50 |
| 45 mol % | 86 | 0.219 | -48 |

To achieve the desired properties in applications of archaeal lipids, chemically modified (semi-synthetic) archaeal lipids are often used⁹. In contrast, the archaeal lipids from *Aeropyrum pernix* K1 used in the formulations of this invention are unmodified. The thermostability of archaeal lipids can also facilitate the sterilization process of the delivery system.

Due to the aerobic nature of *A. pernix* K1, the biotechnological cultivation process is relatively straightforward, providing an economic advantage. The archaea were grown in 1 L reagent bottles with constant stirring (600 magnet revolutions per minute) at 92 °C with aeration (flow rate of 11 L/h). The growth medium was prepared according to the recipe (Table 3) and adjusted to a pH of 7.0. The archaea were harvested after 40 hours. From the harvested archaea, an appropriate polar lipid extract was prepared using established methods.

**Table 3: Composition of the growth medium for cultivating Aeropyrum pernix K1.**

| **GROWTH MEDIUM for *Aeropyrum pernix*** | |
|---|---|
| REAGENT | AMOUNT (/L) |
| Aquarium salt | 32.64 g |
| Pepton (casein) | 6g |
| Yeast extract | 0.96 g |
| Sodium thiosulfate | 0.96 g |
| HEPES (4-(2-hydroxyethyl)-1-piperazineethanesulfonic acid) | 4.77 g |

In one embodiment, the process for preparing liposomal formulations-specifically, small unilamellar liposomes (SUV) and large unilamellar liposomes (LUV)-involves a lipid bilayer in the liposomal formulations that, in addition to phospholipids and cholesterol, includes various proportions of archaeal lipids. The preparation includes the creation of multilamellar vesicles (MLV), which are a precursor in the production of SUV and LUV, and the colloidal suspension of multilamellar liposomes is prepared using the thin film method.

To prepare the colloidal suspension of multilamellar liposomes, a solution of a phospholipid and cholesterol mixture, dissolved in a chloroform and methanol mixture, is prepared. A calculated amount of archaeal lipids dissolved in chloroform is then added to the mixture. Using a rotary evaporator, the solvents are evaporated from the combined mixture until a dry film is obtained. This dry film is hydrated with a buffer to reach the desired final lipid concentration, preferably 5 mg/mL. The formation of liposomes is facilitated by alternating cycles of vortexing and sonication in an ultrasonic bath, resulting in a colloidal suspension of multilamellar liposomes.

From the prepared colloidal suspension of multilamellar liposomes, a colloidal solution of small unilamellar liposomes (SUV) with diameters up to 100 nm is prepared by sonication. Preferably, sonication is performed for 30 minutes at a frequency of 22 kHz and an amplitude of 35%, with 10-second on-off cycles.

From the prepared colloidal suspension of multilamellar liposomes, a colloidal solution of large unilamellar liposomes (LUV) with diameters of 100-1000 nm is prepared by extrusion through a membrane. The pore size of the membrane through which the colloidal suspension of multilamellar liposomes is extruded determines the desired size of the large unilamellar liposomes. To ensure the morphological properties of the large liposomes, it is recommended to perform at least 21 passes (extrusions) through the membrane.

In one embodiment, the process for preparing giant unilamellar liposomes (GUV), where the lipid bilayer of the liposomal formulations includes various proportions of archaeal lipids in addition to phospholipids and cholesterol, is carried out using the gel swelling method. The process involves preparing a dried polymer film (e.g., agarose, polyvinyl alcohol...) and filling the GUV with sucrose, followed by preparing a suspension in an equimolar glucose solution. This greatly facilitates the use of microscopic techniques for GUV analysis. In our case, we chose high molecular weight polyvinyl alcohol as the polymer because it does not contaminate the sample. The process includes preparing a dried polyvinyl alcohol film with sucrose, whose surface is confined by an O-ring. The desired lipid formulation mixture, dissolved in chloroform, is applied to the dried film to create a secondary film. The addition of sucrose buffer causes the lipid film to swell and form GUV. After 30 minutes of swelling, the suspension is carefully pipetted into a buffer with an equimolar concentration of glucose (relative to sucrose).

In another embodiment, the process for preparing giant unilamellar liposomes (GUV) is carried out using the electroformation method.

In some embodiments, the process for preparing liposomal formulations-specifically, unilamellar liposomes of the specified sizes-can be carried out by injecting the desired lipid suspension into the aqueous phase, using emulsification methods, or by the proliposome method. The embodiment procedures demonstrate that the formation of smaller liposomes (<100 nm) is significantly facilitated compared to conventional formulations of sphingomyelin and cholesterol. With the addition of archaeal lipids, smaller structures are obtained without the use of co-solvents (which would otherwise need to be removed in subsequent processing, a step we entirely avoid in our case).

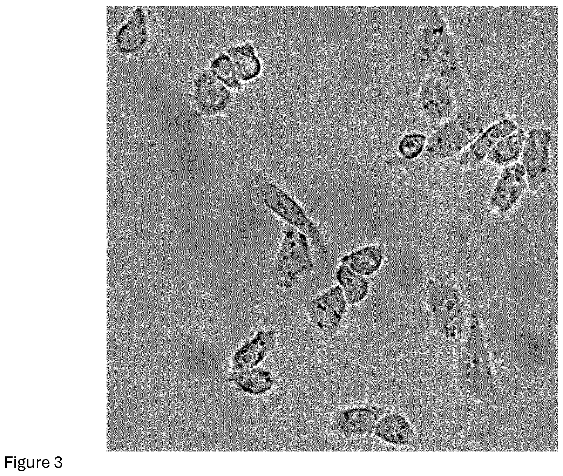

Figure 4 shows the improved morphological properties of large unilamellar vesicles. In liposomes with added archaeal lipids (B), a significantly reduced degree of mutual adhesion is observed, which is due to the reduced zeta potential of the liposomes. Additionally, liposomes with added archaeal vesicles (B) exhibit a more homogeneous size distribution. Alongside these improved morphological properties, the uptake of liposomes by cells is also enhanced, as seen in Figure 3.

The embodiment examples include the preparation of liposomes of various sizes and demonstrate:
- The lipid composition of liposomes based on egg sphingomyelin and cholesterol (at a fixed molar ratio of 6:4), where the key innovation is the addition of various proportions of archaeal lipids (in examples ranging from 2-45 mol %).
- Preparation of small unilamellar vesicles (SUV) with diameters up to 100 nm.
- Preparation of large unilamellar vesicles (LUV) with diameters of 100-1000 nm.
- Preparation of giant unilamellar vesicles (GUV) with diameters over 1 µm.
- Improved zeta potential of the new SUV and LUV liposomes (≤ -30 mV to achieve colloidal stability of the suspensions).
- Increased membrane fluidity of the new SUV liposomes (reduced lipid order parameter-S).
- Improved long-term stability of the new LUV liposomes when stored at 4 °C and even at elevated temperatures (45 °C).
- High stability even at higher temperatures (virtually no release of calcein from vesicles within the temperature range of 20-98 °C; heating rate of 1 °C/min).
- Preparation of multilamellar vesicles (MLV), which serve as a precursor in the production of SUV and LUV.

### Embodiment Examples

### Example 1

A) Preparation of a colloidal suspension of multilamellar vesicles using the thin film method
   A mixture of egg sphingomyelin and cholesterol (molar ratio 6:4), dissolved in a chloroform and methanol mixture (volume ratio 7:3), is prepared. In a 10 mL rotary evaporator flask, a calculated amount of archaeal lipids dissolved in chloroform is added to the egg sphingomyelin and cholesterol mixture (the total lipid mass should not exceed 15 mg). The final molar ratios of lipids in various formulations are shown in Table 1. Chloroform evaporation on the rotary evaporator is performed at 50 °C, 200 mbar, and 90 rpm. For complete drying, the pressure is reduced to a minimum (approximately 16 mbar; drying lasts at least 3 hours). The dry film is hydrated with PBS buffer (pH 7.4) or 20 mM HEPES (pH 7.0) to a final lipid concentration of 5 mg/mL. Vesicle formation is facilitated by three cycles of vortexing (10 seconds) and sonication in an ultrasonic bath (30 seconds at maximum power), resulting in a colloidal suspension of multilamellarvesicles.
B) Preparation of smaller vesicles (approximately 80 nm in diameter) by sonication
   The prepared colloidal suspension of multilamellar vesicles is diluted with the selected buffer to 1 mg/mL. An ice bath is prepared, and a 2 mL Eppendorf tube is filled with 1-1.5 mL of the vesicle colloidal suspension, which is then sonicated for 30 minutes at a frequency of 22 kHz and an amplitude of 35%, with 10-second on-off cycles (device: Vibracell Ultrasonic Disintegrator VCX 750, Sonics and Materials, Newtown, MA, USA). The sonicated vesicles are centrifuged for 10 minutes at 12,000 RCF to remove any residues from the ultrasonic probe.

### Example 2

### A) Preparation of large vesicles (approximately 145 nm in diameter) by extrusion

Large vesicles are prepared using an extrusion system from Avanti Polar Lipids (Alabaster, USA) with a membrane pore size of 100 nm (for an average vesicle diameter of 145 nm) or 400 nm (average vesicle diameter of 200 nm). A colloidal suspension of multilamellar vesicles, prepared in the same manner as in Example 1 - Step A, is used. The prepared colloidal suspension of multilamellar vesicles is diluted with the selected buffer to 1 mg/mL. A glass syringe (V = 1 mL) with a Teflon plunger is filled with the multilamellar vesicle suspension, and the suspension is extruded through the membrane. To achieve the required size and low polydispersity of large vesicles, at least 21 passes (extrusions) through the membrane are required.

### Example 3

### A) Preparation of giant vesicles (approximately 1-100 µm in Diameter) using gel swelling

A 5% (w/w) solution of polyvinyl alcohol (PVA) (molecular weight 145.000 Da) is prepared in a 280 mM sucrose solution (γ = 95.84 g/L). The mixture is stirred at 90 °C to dissolve the PVA. A glass slide (approximately 30 mm in diameter) is coated with 100-300 µL of the PVA solution and then dried for 30 minutes at 50 °C. A 10-20 µL lipid solution of the desired composition, dissolved in chloroform (1 mg/mL), is evenly applied to the dried PVA film. The lipids are then dried under vacuum for 30 minutes. A chamber with a diameter of 1-2 cm is created (e.g., using an O-ring), and 1 mL of buffer (HEPES or PBS with 280 mM sucrose) is added. After 30 minutes, the suspension of the resulting giant liposomes is carefully pipetted into a buffer with 280 mM glucose.

### REFERENCES

(1) Filipczak, N.; Pan, J.; Yalamarty, S. S. K.; Torchilin, V. P. Recent Advancements in Liposome Technology. Advanced Drug Delivery Reviews 2020, 756, 4-22. https://doi.org/10.1016/j.addr.2020.06.022.
(2) Worsham, R. D.; Thomas, V.; Farid, S. S. Potential of Continuous Manufacturing for Liposomal Drug Products. Biotechnol. J. 2019, 14 (2), 1700740. https://doi.org/10.1002/biot.201700740.
(3) Gmajner, D.; Ota, A.; Šentjurc, M.; Ulrih, N. P. Stability of Diether C25,25 Liposomes from the Hyperthermophilic Archaeon Aeropyrum Pernix K1. *Chemistry and Physics of Lipids* **2011,** *164* (3), 236-245. https://doi.org/10.1016/j.chemphyslip.2011.01.005.
(4) Gmajner, D.; Grabnar, P.A.; Žnidarič, M. T.; Štrus, J.; Šentjurc, M.; Ulrih, N. P. Structural Characterization of Liposomes Made of Diether Archaeal Lipids and Dipalmitoyl-L-α-Phosphatidylcholine. *Biophysical Chemistry* **2011,** *158* (2-3), 150-156. https://doi.org/10.1016/j.bpc.2011.06.014.
(5) Markelc, B.; Napotnik, T. B.; Ota, A.; Cemazar, M.; Ulrih, N. P.; Miklavcic, D.; Sersa, G. Archaeosomes Prepared from Aeropyrum Pernix K1 Lipids as an In Vivo Targeted Delivery System for Subsequent Nanosecond Electroporation. In *6th European Conference of the International Federation for Medical and Biological Engineering;* Lacković, I., Vasic, D., Eds.; IFMBE Proceedings; Springer International Publishing: Cham, 2015; Vol. 45, pp 561-564. https://doi.org/10.1007/978-3-319-11128-5_140.
(6) Napotnik, T. B.; Valant, J.; Gmajner, D.; Passamonti, S.; Miklavčič, D.; Ulrih, N. P. Cytotoxicity and Uptake of Archaeosomes Prepared from *Aeropyrum Pernix* Lipids. *Hum Exp Toxicol* **2013,** *32* (9), 950-959. https://doi.org/10.1177/0960327113477875.
(7) Bulbake, U.; Doppalapudi, S.; Kommineni, N.; Khan, W. Liposomal Formulations in Clinical Use: An Updated Review. Pharmaceutics 2017, 9 (4), 12. https://doi.org/10.3390/pharmaceutics9020012.
(8) Silverman, J. A.; Deitcher, S. R. Marqibo® (Vincristine Sulfate Liposome Injection) Improves the Pharmacokinetics and Pharmacodynamics of Vincristine. Cancer Chemother Pharmacol 2013, 71 (3), 555-564. https://doi.org/10.1007/s00280-012-2042-4.
(9) Sprott, G.; Dicaire, C.; Fleming, L.; Patel, G. Stability of Liposomes Prepared from Archaeobacterical Lipids and Phosphatidylcholine Mixtures. Cells and Materials 1996, 6 (1).

## Claims

1. A liposomal formulation, wherein the liposomal formulation is a small unilamellar liposome, a large unilamellar liposome, or a giant unilamellar liposome, **characterized in that** the lipid bilayer of the liposomal formulation consists of phospholipids, cholesterol, and archaeal lipids, wherein the molar fraction of phospholipids is between 40 and 80 mol %, the molar fraction of cholesterol is between 20 and 60 mol %, and the molar fraction of the polar lipid extract from the archaeon *Aeropyrum pernix* K1 is between 0.5 and 50 mol %.

2. The liposomal formulation according to claim 1, **characterized in that** the phospholipid is selected from phosphatidylcholine, phosphatidylserine, phosphatidylethanolamine, or sphingomyelin, with acyl side chains containing 8 to 24 carbon atoms.

3. The liposomal formulation according to claim 1 or 2, **characterized in that** the phospholipid is sphingomyelin, with acyl side chains containing 8 to 24 carbon atoms, where the sphingomyelin may be of natural origin, such as egg or brain sphingomyelin, or synthetic.

4. The liposomal formulation according to claims 1 to 3, **characterized in that** the polar lipid extract from the archaeon *Aeropyrum pernix* K1 consists of two fractions of polar lipids, specifically 91 mol % of 2,3-di-O-sesterterpanyl-*sn-*glycerol-1-phospho-1'-(2'-O-α-D-glucosyl)-myo-inositol (C_{25,25}-archaetidyl(glucosyl)inositol) and 9 mol % of 2,3-di-O-sesterterpanil-*sn*-glycerol-1-phospho-myo-inositol (C_{25,25}-archaetidylinositol).

5. A process for preparing liposomal formulations, wherein the liposomal formulations are small unilamellar liposomes, **characterized in that** the process includes:
- preparing a colloidal suspension of multilamellar liposomes using the thin film method;
- from the prepared colloidal suspension of multilamellar liposomes, a colloidal solution of small unilamellar liposomes (SUV) with a diameter up to 100 nm is prepared by sonication, wherein the sonication is preferably carried out for 30 minutes at a frequency of 22 kHz and an amplitude of 35%, with 10-second on-off cycles.

6. The process for preparing liposomal formulations according to claim 5, **characterized in that** the process is carried out by injecting the desired lipid suspension formulation into the aqueous phase, using emulsification methods or the proliposome method.

7. A process for preparing liposomal formulations, wherein the liposomal formulations are large unilamellar liposomes, **characterized in that** the process includes:
- preparing a colloidal suspension of multilamellar liposomes using the thin film method;
- from the prepared colloidal suspension of multilamellar liposomes, a colloidal solution of large unilamellar liposomes (LUV) with a diameter from 100 to 1000 nm is prepared by extrusion through a membrane, with at least 21 passes through the membrane.

8. The process for preparing liposomal formulations according to claim 7, **characterized in that** the process is carried out by injecting the desired lipid suspension formulation into the aqueous phase, using emulsification methods or the proliposome method.

9. A process for preparing liposomal formulations, wherein the liposomal formulations are giant unilamellar liposomes, **characterized in that** the process includes:
- preparing a dried alcohol film with sucrose, with its surface confined by an O-ring;
- applying a mixture of the desired lipid formulation, dissolved in chloroform, onto the dried film to create a secondary film;
- adding a sucrose buffer, which causes the lipid film to swell and thereby form giant unilamellar liposomes (GUV) in suspension;
- pipetting the GUV suspension into a buffer with equimolar glucose.

10. The process for preparing liposomal formulations according to claim 9, **characterized in that** the process is carried out by the electroformation method.

11. The process for preparing liposomal formulations according to claim 9, **characterized in that** the process is carried out by injecting the desired lipid suspension formulation into the aqueous phase, using emulsification methods or the proliposome method.

12. The liposomal formulations according to claims 1 to 4, wherein the liposomal formulations are small or large unilamellar liposomes, for the delivery of active ingredients to a target site.
